# EUROPEAN PATENT SPECIFICATION

(11) **EP 4 642 518 B1**
(45) Date of publication and mention of the grant of the patent: **18.11.2026**
(21) Application number: 23840806.6
(22) Date of filing: 28.12.2023
(51) Int. Cl.: A61N 1/36, A61N 1/40

(54) **REDUCING ELECTROSENSATION WHILE TREATING A SUBJECT USING ALTERNATING ELECTRIC FIELDS BY INCREASING THE NUMBER OF STEPS IN A RAMP-UP PORTION OF A WAVEFORM**
REDUZIERUNG DER ELEKTROSENSIERUNG WÄHREND DER BEHANDLUNG EINES PATIENTEN MIT ELEKTRISCHEN WECHSELFELDERN DURCH ERHÖHUNG DER SCHRITTZAHL IN EINEM RAMPENABSCHNITT EINER WELLENFORM
RÉDUCTION DE L'ÉLECTROSENSATION LORS DU TRAITEMENT D'UN SUJET À L'AIDE DE CHAMPS ÉLECTRIQUES ALTERNATIFS PAR AUGMENTATION DU NOMBRE D'ÉTAPES DANS UNE PARTIE ASCENDANTE D'UNE FORME D'ONDE

(30) Priority: 29.12.2022 US 202263435967 P
(43) Date of publication of application: 05.11.2025
(73) Proprietor: Novocure GmbH, 6340 Baar (CH)
(72) Inventor: WASSERMAN, Yoram, 31905 Haifa (IL); BEN-TOV KUPERBERG, Mor, 31905 Haifa (IL); SHTOTLAND, Michael, 31905 Haifa (IL)
(74) Representative: Carpmaels & Ransford LLP
(86) International application number: PCT/IB2023/063297
(87) International publication number: WO 2024/141958

(56) References cited:
- CN-A- 114 177 528
- US-A1- 2022 161 028

## Description

### BACKGROUND

Tumor Treating Fields (TTFields) therapy is a proven approach for treating tumors using alternating electric fields at frequencies between 50 kHz and 1 MHz (e.g., 150-200 kHz). In the prior art Optune^{®} system, TTFields are delivered to patients via four transducer arrays that are placed on the patient's skin near the tumor. The transducer arrays are arranged in two pairs, with one pair of transducer arrays positioned to the left and right of the tumor, and the other pair of transducer arrays positioned anterior and posterior to the tumor. Each transducer array is connected via a multi-wire cable to an AC signal generator. The AC signal generator (a) sends an AC current through the anterior/posterior (A/P) pair of transducer arrays for 1 second, which induces an electric field with a first direction through the tumor; then (b) sends an AC current through the left/right (L/R) pair of arrays for 1 second, which induces an electric field with a second direction through the tumor; then repeats steps (a) and (b) for the duration of the treatment. Each transducer array includes a plurality (e.g., between 9 and 30) of electrode elements.

Alternating electric fields can also be used to treat medical conditions other than tumors. For example, as described in U.S. Patent No. 10,967,167, alternating electric fields can be used to increase the permeability of the blood brain barrier (BBB) so that, e.g., chemotherapy drugs can reach the brain.

When treating a subject using alternating electric fields, higher amplitudes are strongly associated with higher efficacy of treatment. However, as the amplitude of the alternating electric field increases, and/or as the frequency of the alternating electric field decreases (e.g., to the vicinity of 100 kHz), some subjects experience an electrosensation effect. This electrosensation could be, for example, a vibratory sensation, paresthesia, and/or a twitching or contraction sensation of muscle fibers, or a flicker of light in the eyes (phosphene). Electrosensation may discourage some subjects from continuing their treatment using alternating electric fields. Furthermore, electrosensation can limit the amplitude of the alternating electric fields that can comfortably be applied to the given subject, which in turn can limit the efficacy of the treatment.

US 2022/0161028 A1 provides an alternating electric field that may be applied to a target region in a living body or to cells in vitro. The alternating electric field is applied during a first interval of time that is at least 1 hour long. The first interval of time includes a plurality of (e.g., 10) non-overlapping sub-intervals of time per hour. In each of the sub-intervals of time, (a) the alternating electric field has a frequency between 50 kHz and 1 MHz (e.g., 50-500 kHz, or 100-300 kHz), (b) the alternating electric field has a respective peak intensity of at least 0.1 V/cm in at least a portion of the target region, and (c) the alternating electric field remains at the respective peak intensity less than 75% the time (e.g., 25% or 33% of the time).

### SUMMARY OF THE INVENTION

The invention is directed to a first apparatus that comprises a signal generator and a controller. The signal generator has at least one control input, and is configured to generate a first AC output at a frequency between 50 kHz and 1 MHz. The first AC output has an amplitude that depends on a state of the at least one control input. The controller is configured to send a first set of control signals to the at least one control input during each of a plurality of first intervals of time per hour. The first set of control signals during each of the first intervals of time is configured to increase an amplitude of the first AC output in a stepwise manner during a first portion of the first interval of time, and the stepwise increase during the first portion of the first interval of time includes at least 170 steps.

In some embodiments of the first apparatus, each of the at least 170 steps has a height of less than 1 V. In some embodiments of the first apparatus, during each of the first intervals of time, the stepwise increase during the first portion of the first interval of time includes at least 200 steps.

In some embodiments of the first apparatus, during each of the first intervals of time, the first AC output has an amplitude that remains substantially constant during a second portion of the first interval of time that comes after the first portion of the first interval of time. Optionally, in these embodiments, during each of the first intervals of time, the first AC output has an amplitude that decreases stepwise during a third portion of the first interval of time that comes after the second portion of the first interval of time.

In some embodiments of the first apparatus, the plurality of first intervals of time includes at least 100 first intervals of time that all occur within one hour. In some embodiments of the first apparatus, the first AC output has a frequency between 100 kHz and 500 kHz.

In some embodiments of the first apparatus, the signal generator is further configured to generate a second AC output at a frequency between 50 kHz and 1 MHz, and the second AC output has an amplitude that depends on a state of the at least one control input. The controller is further configured to send a second set of control signals to the at least one control input during each of a plurality of second intervals of time per hour. The second set of control signals during each of the second intervals of time is configured to increase an amplitude of the second AC output in a stepwise manner during a first portion of the second interval of time, and the stepwise increase during the first portion of the second interval of time includes at least 170 steps.

Optionally, in the embodiments described in the previous paragraph, during each of the first intervals of time, the stepwise increase during the first portion of the first interval of time includes at least 200 steps. And during each of the second intervals of time, the stepwise increase during the first portion of the second interval of time includes at least 200 steps.

In some embodiments of the first apparatus, the signal generator is further configured to generate a second AC output at a frequency between 50 kHz and 1 MHz, and the second AC output has an amplitude that depends on a state of the at least one control input. The controller is further configured to send a second set of control signals to the at least one control input during each of a plurality of second intervals of time per hour. The second set of control signals during each of the second intervals of time is configured to increase an amplitude of the second AC output in a stepwise manner during a first portion of the second interval of time, and the stepwise increase during the first portion of the second interval of time includes at least 170 steps. During each of the first intervals of time, the first AC output has an amplitude that remains substantially constant during a second portion of the first interval of time that comes after the first portion of the first interval of time. During each of the second intervals of time, the first AC output has an amplitude that remains substantially constant during a second portion of the second interval of time that comes after the first portion of the second interval of time. The plurality of first intervals of time includes at least 100 first intervals of time that all occur within one hour, and the plurality of second intervals of time includes at least 100 second intervals of time that all occur within the one hour.

### BRIEF DESCRIPTION OF THE DRAWINGS

FIG. 1 depicts the AC output amplitudes in two channels of the prior art Optune^{®} system.
FIG. 2 is a block diagram of a system for driving a set of transducer arrays with AC voltage signals in which the ramp-up in amplitude of the AC outputs includes at least 170 steps.
FIG. 3 is a schematic representation of a signal that is applied to a set of transducer arrays with a peak amplitude of 100 V, with 100 steps in the ramp-up period.
FIG. 4 is a schematic representation of a signal that is applied to a set of transducer arrays with a peak amplitude of 200 V, with 100 steps in the ramp-up period.
FIG. 5 is a schematic representation of a signal that is applied to a set of transducer arrays with a peak amplitude of 200 V, with 200 steps in the ramp-up period. This signal can be used to ameliorate electrosensation.

Various embodiments are described in detail below with reference to the accompanying drawings, wherein like reference numerals represent like elements.

### DESCRIPTION OF THE PREFERRED EMBODIMENTS

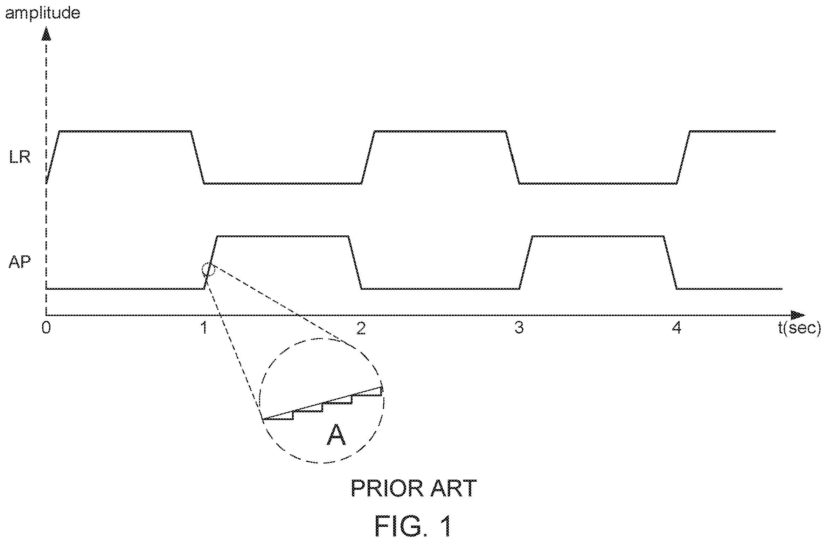

FIG. 1 depicts the AC output amplitudes of the L/R channel and the A/P channel in the prior art Optune^{®} system. Notably, when the signal to either the A/P or L/R transducer arrays is turned on during any given one-second interval, the amplitude of the AC voltage does not jump immediately to its peak value. Instead, the amplitude of the AC voltage ramps up from zero to its peak during a window of time that is nominally 100 ms and never exceeds 160 ms. Similarly, when the signal is turned off during any given one-second interval, the amplitude of the AC voltage ramps down from its peak to zero during another window of time that is nominally 100 ms and never exceeds 160 ms.

In the prior art Optune^{®} system, the ramping up and down of the amplitude was implemented by updating a control word within the AC signal generator at regular 1 ms intervals during the ramp up and ramp down portions of the waveform. Thus, although the main portion of FIG. 1 depicts the ramp-up portion of the waveform as being smooth, zooming in on the waveform reveals that the amplitude actually resembles a staircase, as depicted in inset A of FIG. 1. And because the ramp-up portion of the waveform in Optune^{®} never exceeds 160 ms, the staircase-shaped ramp-up in amplitude never exceeds 160 steps.

Without being bound by this theory, electrosensation is believed to originate from interactions between the alternating electric fields and nerve cells or fibers (i.e., neurons or axons) that are positioned near or adjacent to the transducer arrays. In the prior art Optune^{®} system, electrosensation was not a major problem. But when the inventors modified an Optune^{®} system to operate at higher amplitudes or lower frequencies (but unchanged in all other relevant respects), electrosensation became much more problematic.

This application describes approaches for ameliorating electrosensation in systems that generate alternating electric fields (e.g., TTFields) by increasing the number of steps in the ramp-up portion of the waveform.

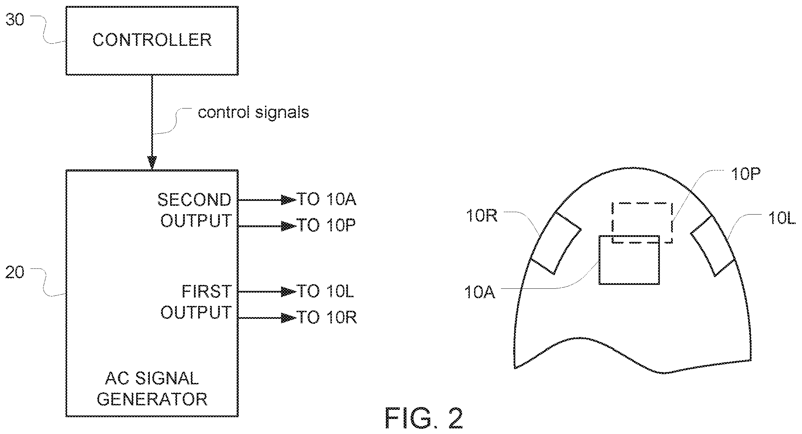

FIG. 2 is a block diagram of a system for driving a set of transducer arrays with AC voltage signals in which the amplitude of the AC voltage can be controlled. The system includes an AC signal generator 20 that is designed to generate first and second AC outputs at a frequency between 50 kHz and 10 MHz (e.g., 50 kHz-1 MHz, 50-500 kHz, 75-300 kHz, or 150-250 kHz). When the system is used to apply TTFields to a person's body (as shown in FIG. 2), the first AC output is applied across a first pair of transducer arrays 10L and 10R that are positioned to the left and right of the tumor; and the second AC output is applied across a second pair of transducer arrays 10A and 10P that are positioned anterior and posterior to the tumor.

When the AC signal generator 20 applies a voltage between transducer arrays 10L, 10R, an alternating electric field is induced through the target region with field lines that run generally from left to right. And when the AC signal generator 20 applies a voltage between transducer arrays 10A, 10P, an alternating electric field is induced through the target region with field lines that run generally from front to back. The frequency of the alternating electric field will match the frequency of the AC signal generator 20. The electrode elements in the transducer arrays 10 can be capacitively-coupled electrode elements (i.e., electrode elements that include a thin dielectric layer that contacts the subject's body) or conductive electrode elements (i.e., electrode elements that include a conductive surface that contacts the subject's body).

In some embodiments, the voltage generated by the AC signal generator 20 is sufficient to induce an electric field of at least 1 V/cm in at least a portion of the cells. For example, in some embodiments, the voltage generated by the AC signal generator 20 is sufficient to induce an electric field of at least 2 V/cm, at least 3 V/cm, or at least 5 V/cm in at least a portion of the cells. In some embodiments, the voltages generated by the AC signal generator 20 is sufficient to induce an electric field of 1-10 V/cm, 2-10 V/cm, 5-10 V/cm, or 2-20 V/cm in at least a portion of the cells.

As in the prior art Optune^{®} system, (a) the first AC output is applied to the L/R transducer arrays for a period of time; (b) the second AC output is applied to the A/P transducer arrays for a period of time; and the two-step sequence (a) and (b) is repeated for the duration of the treatment. But the way the AC voltage ramps up from zero to its peak in the FIG. 2 embodiment differs from the way the AC voltage ramps up from zero to its peak in Optune^{®}. And this difference is responsible for an amelioration of the electrosensation that is experienced by the subject, as described in more detail below.

The AC signal generator 20 is configured to generate first and second AC outputs with amplitudes that depend on a state of at least one control input. A controller 30 rapidly sends sequential control signals (e.g., at a rate of 1 control signal per ms) to the at least one control input to control the output amplitude of the AC signal generator 20 as described below. Note that although FIG. 2 depicts the controller 30 and the AC signal generator 20 as two distinct blocks, those two blocks may be integrated into a single hardware device.

The details of the construction of the controller 30 and the nature of the control signals will depend on the design of the AC signal generator 20. In one example, the design of the AC signal generator 20 is similar to the AC signal generator described in US patent 9,910,453. This particular AC signal generator has two output channels (i.e., a first channel for L/R and a second channel for A/P). The instantaneous AC output voltage on either channel depends on the instantaneous output voltage of a DC-DC converter, and the output voltage of that DC-DC converter is controlled by writing control words to a digital-to-analog converter (DAC), e.g., at a rate of 1 control signal per ms.

The controller 30 in FIG. 2 can therefore implement any desired rate of increase of the first and second outputs of the AC signal generator 20, with any desired pattern, by sequentially sending appropriate control words to the DAC in the AC signal generator 20.

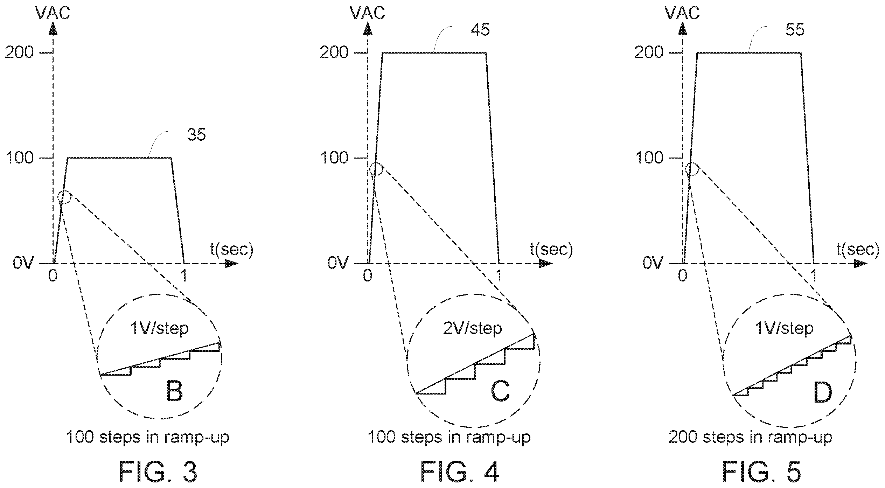

Assume, for example, that a given patient does not experience electrosensation when AC signals that are similar to the ones used in the prior art Optune system are applied to the subject's body with a peak amplitude of 100 V using the left and right transducer arrays 10 L and 10 R. Signal 35 depicted in FIG. 3 is an example of a single instance of such a signal, and this signal 35 is generated repeatedly (e.g., once every two seconds). In signal 35, the amplitude ramps up from 0 VAC to 100 VAC during a 100 ms interval that is divided into 100 equally-spaced steps (i.e., 1 ms per step), then remains at 100 VAC for 800 ms, and then ramps down from 100 VAC to 0 VAC during the next 100 ms interval. In this example, the height of each step in the ramp-up portion of the waveform is 1 V per step.

As noted above, when treating a subject using alternating electric fields, higher amplitudes are strongly associated with higher efficacy of treatment. In the prior art Optune^{®} system, the amplitude of the AC voltage that could be applied to a given subject's body was usually limited by thermal considerations. More specifically, because the Optune^{®} transducer arrays heat up when the amplitude of the AC voltage that is applied to those transducers increases, a safety temperature threshold (e.g., 39° C) is eventually reached. And that temperature threshold has traditionally been the limiting factor that prevented Optune^{®} from operating at higher amplitudes.

Let us now perform a thought experiment to analyze what would happen if the thermal considerations have been overcome to a point where the amplitude of the AC signals that are applied to the same subject's body could be doubled, without making any other changes to the waveform. Signal 45 depicted in FIG. 4 is an example of such a signal. More specifically, in signal 45, the amplitude ramps up from 0 VAC to 200 VAC during a 100 ms interval that is divided into 100 equally-spaced steps (i.e., 1 ms per step), then remains at 200 VAC for 800 ms, and then ramps down from 200 VAC to 0 VAC during the next 100 ms interval.

If the doubled-amplitude signal 45 is applied to a subject's body, there is a high likelihood that the subject will experience electrosensation. In this situation, electrosensation becomes the factor that prevents the system from operating at higher voltages (because we are assuming that thermal considerations are not a limiting factor in this thought experiment). This application describes approaches for ameliorating electrosensation so that the system can operate at higher voltages, which will increase the amplitude of the alternating electric fields, which will in turn increase the efficacy of the treatment.

Notably, with the doubled-amplitude signal 45, the height of each step in the ramp-up portion of the waveform is 2 V per step. And the inventors have found that the increase in the height of each step (i.e., from 1 V to 2 V) during the ramp-up portion of the waveform makes a much more significant contribution to electrosensation than the increase in overall amplitude (i.e., from 100 VAC to 200 VAC) during the 800 ms middle portion of the waveform.

The inventors have further determined that the electrosensation that was introduced by doubling the amplitude of the waveform can be ameliorated by increasing the number of steps that are used during the ramp-up portion of the waveform to the point where the step height gets small enough so that electrosensation is no longer a problem. For example, if the number of steps during a ramp-up from 0 V to 200 V is increased from 100 steps to 200 steps, as depicted for signal 55 in FIG. 5, the height of each step will drop from 2 V per step to 1 V per step, which will often be sufficient to ameliorate the electrosensation. If electrosensation is not ameliorated for a particular patient, the number of steps can be increased further (e.g., to 250 steps, in which case the height of each step will drop down to 0.8 V per step). In another example, if the increase in amplitude is smaller (e.g., an increase from 100 V to 170 V), a smaller increase in the number of steps (e.g., to 170 steps) may be sufficient to ameliorate the electrosensation.

In some embodiments, the duration and height of each individual step remains the same no matter how many steps are included within the ramp-up period. In these embodiments, if we assume that the duration of each step is 1 ms and the height of each step is 1 V, a ramp-up from 0 V to 100 V will take 100 ms, and a ramp-up from 0 V to 200 V will take 200 ms. Thus, in these embodiments, the overall duration of each ramp-up period will depend on the number of steps. In these embodiments, the number of steps may be, but are not limited to, at least 170 steps, at least 200 steps, at least 250 steps, at least 300 steps, at least 350 steps, at least 400 steps, at least 450 steps, at least 500 steps, at least 750 steps, at least 1000 steps, or greater than 1000 steps. Or, for example, the number of steps may be between 200 steps and 10,000 steps, between 200 steps and 1,000 steps, between 200 steps and 750 steps, or between 200 steps and 500 steps.

In other embodiments, the number of steps in each ramp-up period is increased to a fixed number that is higher than the prior art (e.g., increased to 170 or 200 steps), and the duration of each step remains the same as in the prior art (e.g., 1 ms/step). For example, each ramp-up period may have a fixed number of steps that is at least 170 steps, at least 200 steps, at least 250 steps, at least 300 steps, at least 350 steps, at least 400 steps, at least 450 steps, at least 500 steps, at least 750 steps, at least 1000 steps, or greater than 1000 steps. Or, for example, each ramp-up period may have a fixed number of steps that is between 200 steps and 10,000 steps, between 200 steps and 1,000 steps, between 200 steps and 750 steps, or between 200 steps and 500 steps. In these embodiments, the height of each step will depend on the overall height of the ramp. For example, if 200 steps are used in a ramp-up from 0 V to 100 V, the height of each step would be 0.5 V. But if the same 200 steps are used in a ramp-up from 0 V to 200 V, the height of each step would be 1 V. Of course, when 200 steps are used and each step has a duration of 1 ms, the overall duration of the ramp-up period will be longer than the ramp-up periods of the prior art.

In still other embodiments, the number of steps in each ramp-up period is increased to a fixed number that is higher than the prior art (e.g., increased to 170 or 200 steps), and the duration of each step can be smaller (e.g., 0.5 ms) or larger (e.g., 2-10 ms) than in the example described above for signal 55. For example, each ramp-up period may have a fixed number of steps that is at least 170 steps, at least 200 steps, at least 250 steps, at least 300 steps, at least 350 steps, at least 400 steps, at least 450 steps, at least 500 steps, at least 750 steps, at least 1000 steps, or greater than 1000 steps. Or, for example, each ramp-up period may have a fixed number of steps that is between 200 steps and 10,000 steps, between 200 steps and 1,000 steps, between 200 steps and 750 steps, or between 200 steps and 500 steps. In any of such embodiments, the duration of each step may be, for example, less than 1 ms, less than 0.8 ms, less than 0.6 ms, or less than 0.5 ms. Alternatively, the duration of each step may be, for example, at least 2 ms, at least 3 ms, at least 4 ms, at least 5 ms, at least 10 ms, at least 15 ms, at least 20 ms, at least 25 ms, at least 30 ms, at least 40 ms, at least 50 ms, at least 100 ms, at least 1 s, at least 10 s, at least 30 s, at least 1 min., at least 2 minutes, at least 5 minutes, at least 10 minutes, or at least 20 minutes. The duration of each step may be, for example, between 2 ms and 30 minutes, between 2 ms and 20 minutes, between 2 ms and 10 minutes, between 2 ms and 5 minutes, between 1 ms and 2 minutes, between 2 ms and 1 minutes, between 2 ms and 100 ms, between 2 ms and 50 ms, between 2 ms and 25 ms, or between 2 ms and 10 ms. Any such number or ranges of steps may be combined with any such number or range of duration of step in these embodiments. In this situation, the height of each step will depend on the overall height of the ramp. And of course, the overall duration of the ramp-up period will depend on the duration of each step. For example, when the ramp-up period includes 200 steps and the duration of each step is 0.5 ms, the overall duration of the ramp-up period will be 100 ms. But if the ramp-up period includes 200 steps and the duration of each step is 2 ms, the overall duration of the ramp-up period will be 400 ms.

Signals such as signal 55 (in which the number of steps in the ramp-up period is at least 170) can be generated using systems like the one depicted in FIG. 2, which includes a signal generator 20 and a controller 30. The signal generator 20 has at least one control input, and it is configured to generate a first AC output at a frequency between 50 kHz and 1 MHz (e.g., 100-500 kHz). The amplitude of the first AC output depends on a state of the at least one control input. The controller 30 sends a first set of control signals to the at least one control input during each of a plurality of first intervals of time per hour. In some embodiments, there are 1800 first intervals of time per hour (i.e., 30 per minute), and the duration of each first interval of time is on the order of 1 second. But in other embodiments, the number of first intervals in a given hour could be higher or lower (e.g., 10-60 per minute), and the duration of each first interval of time can be adjusted accordingly. The first set of control signals during each of the first intervals of time is configured to increase an amplitude of the first AC output in a stepwise manner during a first portion (i.e., the ramp-up portion on the left side of signal 55) of the first interval of time. The stepwise increase during the ramp-up portion includes at least 170 steps.

In some embodiments, each of the at least 170 steps has a height of less than 1 V. For example, the step height may be less than 0.9 V, less than 0.8 V, less than 0.7 V, less than 0.5 V, less than 0.3 V, or less than 0.1 V. The step height may be between 0.1 V and 0.5 V, between 0.1 V and 0.7 V, between 0.1 V and 0.9 V, between 0.2 V and 0.8 V, or between 0.5 V and 0.9 V. In some embodiments, during each of the first intervals of time, the stepwise increase during the ramp-up portion includes at least 200 steps (e.g., at least 250 steps, at least 300 steps, at least 350 steps, at least 400 steps, at least 450 steps, at least 500 steps, at least 750 steps, at least 1000 steps, or greater than 1000 steps). In some embodiments, during each of the first intervals of time, the ramp-up portion includes between 200 steps and 10,000 steps, between 200 steps and 1,000 steps, between 200 steps and 750 steps, or between 200 steps and 500 steps.

In some embodiments, during each of the first intervals of time, the first AC output has an amplitude that remains substantially constant (e.g., ±5%) during a second portion of the first interval of time that comes after the first portion of the first interval of time. This second portion corresponds to the plateau at the top of signal 55. Optionally, in these embodiments, during each of the first intervals of time, the first AC output has an amplitude that decreases stepwise during a third portion of the first interval of time that comes after the second portion of the first interval of time. This third portion corresponds to the ramp-down period on the right side of signal 55.

In some embodiments, the plurality of first intervals of time includes at least 100 first intervals of time that all occur within one hour.

In some embodiments (including the embodiment depicted in FIG. 2), the alternating electric field is applied to the subject in two directions in an alternating and repeating sequence (e.g., one second in the L/R direction followed by one second in the A/P, repeated for at least one hour). In these embodiments, the signal generator 20 is further configured to generate a second AC output at a frequency between 50 kHz and 1 MHz, and the second AC output has an amplitude that depends on a state of the at least one control input. And the controller 30 is further configured to send a second set of control signals to the at least one control input during each of a plurality of second intervals of time per hour. The second set of control signals during each of the second intervals of time is configured to increase an amplitude of the second AC output in a stepwise manner during a first portion (i.e., the ramp-up portion) of the second interval of time. The stepwise increase during these ramp-up portions also includes at least 170 steps.

Optionally, in these two-directional embodiments, the ramp-up portion includes at least 170 steps (e.g., 200 steps) during each of the first intervals of time, and the ramp-up portion includes at least 170 steps (e.g., at least 200 steps) during each of the second intervals of time.

Methods for ameliorating electrosensation can use signals like signal 55 depicted in FIG. 5, which can be generated using the system depicted in FIG. 2. In these methods, electrosensation can be ameliorated while applying an electric field to a target region in a living body by applying an alternating electric field having a frequency between 50 kHz and 1 MHz (e.g., 100-500 kHz) to the target region during each of a plurality of intervals of time. During each of the intervals of time, an amplitude of the alternating electric field increases stepwise during a first portion (e.g., the ramp-up portion on the left side of signal 55) of the interval, and the stepwise increase during the first portion of the interval includes at least 170 steps.

Optionally, each of the at least 170 steps has a height of less than 1 V. For example, each of the at least 170 steps may have a height of less than 0.9 V, less than 0.8 V, less than 0.75 V, less than 0.7V, less than 0.6 V, or less than 0.5 V. For example, each of the at least 170 steps may have a height of about 0.9 V, about 0.8 V, about 0.7 V, about 0.6 V, about 0.5 V, about 0.4 V, about 0.3 V, about 0.2 V, or about 0.1 V. Optionally, the stepwise increase during the first portion of the interval includes at least 200 steps during each of the intervals of time. For example, the stepwise increase during the first portion of the interval may include at least 200 steps, at least 250 steps, at least 300 steps, at least 350 steps, at least 400 steps, at least 450 steps, or at least 500 steps.

Optionally, during each of the intervals of time, the alternating electric field has an amplitude that remains substantially constant (e.g., ±5%) during a second portion (e.g., the plateau at the top of signal 55) of the interval that comes after the first portion (e.g., the ramp-up portion) of the interval. Optionally, in these embodiments, during each of the intervals of time, the alternating electric field has an amplitude that decreases stepwise during a third portion (e.g., the ramp-down portion on the right side of signal 55) of the interval that comes after the second portion (e.g., the plateau) of the interval.

Optionally, the plurality of intervals of time includes at least 100 intervals of time that all occur within one hour. Optionally, when the first portion of the interval ends during each of the intervals of time, the alternating electric field has an amplitude of at least 1 V/cm, at least 2 V/cm, at least 3 V/cm, or at least 5 V/cm in at least a portion of the target region. For example, when the first portion of the interval ends during each of the intervals of time, the alternating electric field may have an amplitude of between 1 V/cm and 10 V/cm, between 2 V/cm and 10 V/cm, between 5 V/cm and 10 V/cm, between 2 V/cm and 20 V/cm, or between 1 V/cm and 20 V/cm. In other words, in some embodiments the alternating electric field has an amplitude of at least 1 V/cm (e.g., between 1 V/cm and 10 V/cm, between 2 V/cm and 10 V/cm, between 5 V/cm and 10 V/cm, between 2 V/cm and 20 V/cm or between 1 V/cm and 20 V/cm) during the second portion of each of the intervals of time.

Optionally, the alternating electric field is applied to the target region in a first direction during a first subset of the plurality of intervals of time, and the alternating electric field is applied to the target region in a second direction during a second subset of the plurality of intervals of time. The second direction is offset from the first direction by at least 45°.

Returning to FIG. 2, operation of the anterior/posterior channel is similar to the operation for the left/right channel described above in connection with FIG. 5, except that the two channels are activated in an alternating sequence (e.g., at least 1000 times) and are out of phase. When one channel is active, the other channel is off.

A wide variety of alternative designs for the AC signal generator 20 and the controller 30 can be substituted for the example provided above, as long as the controller 30 has the ability to control the AC signal generator 20. For example, if the AC signal generator is designed to respond to an analog control signal, the controller 30 must generate whatever sequence of analog control signals is needed to cause the AC signal generator 20 to output the desired waveforms. In this situation, the controller 30 could be implemented using a microprocessor or microcontroller that is programmed to write appropriate control words to a digital-to-analog converter, the output of which generates the analog control signals that cause the AC signal generator 20 to generate the desired waveforms. Alternatively, the controller 30 could be implemented using an analog circuit that automatically generates the appropriate sequence of control signals (which are then applied to the control input of the AC signal generator).

In the examples described above, the direction of the alternating electric fields was switched between two directions. But in alternative embodiments the direction of the alternating electric fields may be switched between three or more directions (assuming that additional pairs of transducer arrays are provided). For example, the direction of the alternating electric fields may be switched between three directions, each of which is determined by the placement of its own pair of transducer arrays. In other alternative embodiments, the transducer arrays need not be arranged in pairs. See, for example, the transducer array positioning described in US patent 7,565,205. But regardless of the arrangement of the transducer arrays, ramp-up portions that include at least 170 steps are used every time a given transducer array is activated.

In some anatomic locations, the transducer arrays are not positioned on the subject's skin. Instead, the transducer arrays are implanted into the subject's body (e.g., just beneath the subject's skin) so that application of an AC voltage between the transducer arrays will impose the alternating electric fields in a target region of the subject's body.

Finally, in some anatomic locations, instead of switching the orientation of the alternating electric field back and forth between two or more different directions, an electric field with a constant orientation may be used. Embodiments for use with these locations are similar to the FIG. 2 embodiment, except that the AC signal generator 20 has only a single output (e.g., only the L/R output). In these embodiments, the AC voltage generator is configured to, when initially switched on, ramp its voltage up as described above in connection with FIG. 5, and then either leave its output voltage at a fixed level for the duration of the treatment, or switch the single output on and off repeatedly (e.g., on for 0.1-10 s and off for 0.1-10 s). In the latter situation, each time the AC voltage generator switches back on, it ramps its voltage up as described above in connection with FIG. 5.

While the present invention has been disclosed with reference to certain embodiments, numerous modifications, alterations, and changes falling under the scope of the claims are possible. Accordingly, it is intended that the present invention not be limited to the described embodiments, but that it has the full scope defined by the language of the following claims.

## Claims

1. An apparatus comprising:
a signal generator (20) having at least one control input, wherein the signal generator (20) is configured to generate a first AC output at a frequency between 50 kHz and 1 MHz, the first AC output having an amplitude that depends on a state of the at least one control input; and
a controller (30) configured to send a first set of control signals to the at least one control input during each of a plurality of first intervals of time per hour, wherein the first set of control signals during each of the first intervals of time is configured to increase an amplitude of the first AC output in a stepwise manner during a first portion of the first interval of time, **characterized in that** the stepwise increase during the first portion of the first interval of time includes at least 170 steps.

2. The apparatus of claim 1, wherein each of the at least 170 steps has a height of less than 1 V.

3. The apparatus of claim 1, wherein during each of the first intervals of time, the stepwise increase during the first portion of the first interval of time includes at least 200 steps.

4. The apparatus of claim 1, wherein during each of the first intervals of time, the first AC output has an amplitude that remains substantially constant during a second portion of the first interval of time that comes after the first portion of the first interval of time.

5. The apparatus of claim 4, wherein during each of the first intervals of time, the first AC output has an amplitude that decreases stepwise during a third portion of the first interval of time that comes after the second portion of the first interval of time.

6. The apparatus of claim 1, wherein the plurality of first intervals of time includes at least 100 first intervals of time that all occur within one hour.

7. The apparatus of claim 1, wherein the first AC output has a frequency between 100 kHz and 500 kHz.

8. The apparatus of claim 1,
wherein the signal generator (20) is further configured to generate a second AC output at a frequency between 50 kHz and 1 MHz, the second AC output having an amplitude that depends on a state of the at least one control input; and
wherein the controller (30) is further configured to send a second set of control signals to the at least one control input during each of a plurality of second intervals of time per hour, wherein the second set of control signals during each of the second intervals of time is configured to increase an amplitude of the second AC output in a stepwise manner during a first portion of the second interval of time, wherein the stepwise increase during the first portion of the second interval of time includes at least 170 steps.

9. The apparatus of claim 8, wherein during each of the first intervals of time, the stepwise increase during the first portion of the first interval of time includes at least 200 steps, and
wherein during each of the second intervals of time, the stepwise increase during the first portion of the second interval of time includes at least 200 steps.

10. The apparatus of claim 8, wherein during each of the first intervals of time, the first AC output has an amplitude that remains substantially constant during a second portion of the first interval of time that comes after the first portion of the first interval of time,
wherein during each of the second intervals of time, the first AC output has an amplitude that remains substantially constant during a second portion of the second interval of time that comes after the first portion of the second interval of time,
wherein the plurality of first intervals of time includes at least 100 first intervals of time that all occur within one hour, and
wherein the plurality of second intervals of time includes at least 100 second intervals of time that all occur within the one hour.

## Patentansprüche

1. Einrichtung, umfassend:
einen Signalgenerator (20), der mindestens einen Steuereingang aufweist, wobei der Signalgenerator (20) so konfiguriert ist, dass er eine erste Wechselstromausgabe mit einer Frequenz zwischen 50 kHz und 1 MHz erzeugt, wobei die Wechselstromausgabe eine Amplitude aufweist, die von einem Zustand des mindestens einen Steuereingangs abhängt; und
eine Steuereinheit (30), die so konfiguriert ist, dass sie während jedes einer Vielzahl von ersten Zeitintervallen pro Stunde einen ersten Satz von Steuersignalen an den mindestens einen Steuereingang sendet, wobei der erste Satz von Steuersignalen während jedes der ersten Zeitintervalle so konfiguriert ist, dass er eine Amplitude der ersten Wechselstromausgabe während eines ersten Abschnitts des ersten Zeitintervalls auf schrittweise Art erhöht, **dadurch gekennzeichnet, dass** die schrittweise Erhöhung während des ersten Abschnitts des ersten Zeitintervalls mindestens 170 Stufen einschließt.

2. Einrichtung nach Anspruch 1, wobei jede der mindestens 170 Stufen eine Höhe von weniger als 1 V aufweist.

3. Einrichtung nach Anspruch 1, wobei während jedes der ersten Zeitintervalle die schrittweise Erhöhung während des ersten Abschnitts des ersten Zeitintervalls mindestens 200 Stufen einschließt.

4. Einrichtung nach Anspruch 1, wobei während jedes der ersten Zeitintervalle die erste Wechselstromausgabe eine Amplitude aufweist, die während eines zweiten Abschnitts des ersten Zeitintervalls, der auf den ersten Abschnitt des ersten Zeitintervalls folgt, im Wesentlichen konstant bleibt.

5. Einrichtung nach Anspruch 4, wobei während jedes der ersten Zeitintervalle die erste Wechselstromausgabe eine Amplitude aufweist, die während eines dritten Abschnitts des ersten Zeitintervalls, der auf den zweiten Abschnitt des ersten Zeitintervalls folgt, schrittweise abnimmt.

6. Einrichtung nach Anspruch 1, wobei die Vielzahl von ersten Zeitintervallen mindestens 100 erste Zeitintervalle einschließt, die alle innerhalb einer Stunde auftreten.

7. Einrichtung nach Anspruch 1, wobei die erste Wechselstromausgabe eine Frequenz zwischen 100 kHz und 500 kHz aufweist.

8. Einrichtung nach Anspruch 1,
wobei der Signalgenerator (20) weiter so konfiguriert ist, dass er eine zweite Wechselstromausgabe mit einer Frequenz zwischen 50 kHz und 1 MHz erzeugt, wobei die zweite Wechselstromausgabe eine Amplitude aufweist, die von einem Zustand des mindestens einen Steuereingangs abhängt; und
wobei die Steuereinheit (30), weiter so konfiguriert ist, dass sie während jedes einer Vielzahl von zweiten Zeitintervallen pro Stunde einen zweiten Satz von Steuersignalen an den mindestens einen Steuereingang sendet, wobei der zweite Satz von Steuersignalen während jedes der ersten Zeitintervalle so konfiguriert ist, dass er eine Amplitude der zweiten Wechselstromausgabe während eines ersten Abschnitts des zweiten Zeitintervalls auf schrittweise Art erhöht, wobei die schrittweise Erhöhung während des ersten Abschnitts des zweiten Zeitintervalls mindestens 170 Stufen einschließt.

9. Einrichtung nach Anspruch 8, wobei während jedes der ersten Zeitintervalle die schrittweise Erhöhung während des ersten Abschnitts des ersten Zeitintervalls mindestens 200 Stufen einschließt, und
wobei während jedes der zweiten Zeitintervalle die schrittweise Erhöhung während des ersten Teils des zweiten Zeitintervalls mindestens 200 Stufen einschließt.

10. Einrichtung nach Anspruch 8, wobei während jedes der ersten Zeitintervalle die erste Wechselstromausgabe eine Amplitude aufweist, die während eines zweiten Abschnitts des ersten Zeitintervalls, der auf den ersten Abschnitt des ersten Zeitintervalls folgt, im Wesentlichen konstant bleibt,
wobei die erste Wechselstromausgabe während jedes der zweiten Zeitintervalle eine Amplitude aufweist, die während eines zweiten Abschnitts des zweiten Zeitintervalls, der auf den ersten Abschnitt des zweiten Zeitintervalls folgt, im Wesentlichen konstant bleibt,
wobei die Vielzahl von ersten Zeitintervallen mindestens 100 erste Zeitintervalle einschließt, die alle innerhalb einer Stunde auftreten, und
wobei die Vielzahl von zweiten Zeitintervallen mindestens 100 zweite Zeitintervalle einschließt, die alle innerhalb der einen Stunde auftreten.

## Revendications

1. Appareil comprenant :
un générateur de signaux (20) présentant au moins une entrée de commande, dans lequel le générateur de signaux (20) est configuré pour générer une première sortie CA à une fréquence comprise entre 50 kHz et 1 MHz, la première sortie CA présentant une amplitude dépendant d'un état de la au moins une entrée de commande ; et
un dispositif de commande (30) configuré pour envoyer un premier ensemble de signaux de commande à la au moins une entrée de commande pendant chacun parmi une pluralité de premiers intervalles de temps par heure, dans lequel le premier ensemble de signaux de commande pendant chacun des premiers intervalles de temps est configuré pour augmenter l'amplitude de la première sortie CA de manière progressive pendant une première partie du premier intervalle de temps, **caractérisé en ce que** l'augmentation progressive pendant la première partie du premier intervalle de temps inclut au moins 170 échelons.

2. Appareil selon la revendication 1, dans lequel chacun des au moins 170 échelons présente une hauteur inférieure à 1 V.

3. Appareil selon la revendication 1, dans lequel, pendant chacun des premiers intervalles de temps, l'augmentation progressive pendant la première partie du premier intervalle de temps inclut au moins 200 échelons.

4. Appareil selon la revendication 1, dans lequel, pendant chacun des premiers intervalles de temps, la première sortie CA présente une amplitude qui reste sensiblement constante pendant une deuxième partie du premier intervalle de temps qui suit la première partie du premier intervalle de temps.

5. Appareil selon la revendication 4, dans lequel, pendant chacun des premiers intervalles de temps, la première sortie CA présente une amplitude qui diminue progressivement pendant une troisième partie du premier intervalle de temps qui suit la deuxième partie du premier intervalle de temps.

6. Appareil selon la revendication 1, dans lequel la pluralité de premiers intervalles de temps inclut au moins 100 premiers intervalles de temps qui se produisent tous en l'espace d'une heure.

7. Appareil selon la revendication 1, dans lequel la première sortie CA présente une fréquence comprise entre 100 kHz et 500 kHz.

8. Appareil selon la revendication 1,
dans lequel le générateur de signaux (20) est configuré en outre pour générer une seconde sortie CA à une fréquence comprise entre 50 kHz et 1 MHz, la seconde sortie CA présentant une amplitude qui dépend de l'état de la au moins une entrée de commande ; et
dans lequel le dispositif de commande (30) est configuré en outre pour envoyer un second ensemble de signaux de commande à la au moins une entrée de commande pendant chacun parmi une pluralité de seconds intervalles de temps par heure, dans lequel le second ensemble de signaux de commande pendant chacun des seconds intervalles de temps est configuré pour augmenter l'amplitude de la seconde sortie CA de manière progressive pendant une première partie du second intervalle de temps, dans lequel l'augmentation progressive pendant la première partie du second intervalle de temps inclut au moins 170 échelons.

9. Appareil selon la revendication 8, dans lequel, pendant chacun des premiers intervalles de temps, l'augmentation progressive pendant la première partie du premier intervalle de temps inclut au moins 200 échelons, et
dans lequel, pendant chacun des seconds intervalles de temps, l'augmentation progressive pendant la première partie du second intervalle de temps inclut au moins 200 échelons.

10. Appareil selon la revendication 8, dans lequel, pendant chacun des premiers intervalles de temps, la première sortie CA présente une amplitude qui reste sensiblement constante pendant une deuxième partie du premier intervalle de temps qui suit la première partie du premier intervalle de temps,
dans lequel, pendant chacun des seconds intervalles de temps, la première sortie CA présente une amplitude qui reste sensiblement constante pendant une deuxième partie du second intervalle de temps qui suit la première partie du second intervalle de temps,
dans lequel la pluralité de premiers intervalles de temps inclut au moins 100 premiers intervalles de temps qui se produisent tous en l'espace d'une heure, et
dans lequel la pluralité de seconds intervalles de temps inclut au moins 100 seconds intervalles de temps qui se produisent tous dans l'heure.
